# EUROPEAN PATENT SPECIFICATION

(11) **EP 0 552 829 B1**
(45) Date of publication and mention of the grant of the patent: **21.04.1999**
(21) Application number: 93200062.3
(22) Date of filing: 12.01.1993
(51) Int. Cl.: C07K 14/00, C12Q 1/68, G01N 33/53, G01N 33/564, G01N 33/68

(54) **CENP-B peptide**
CENP-B peptid
Peptide de CENP-B

(30) Priority: 13.01.1992 EP 92200065
(43) Date of publication of application: 28.07.1993
(73) Proprietor: Akzo Nobel N.V., 6824 BM Arnhem (NL)
(72) Inventor: Verheyen, Ronald, 6611 CE Overasselt (NL); van Venrooy, Waltherus Jacobus, 6523 MZ Nijmegen (NL)
(74) Representative: Hermans, Franciscus G.M.

(56) References cited:
- WO-A-91/06572
- JOURNAL OF CELL BIOLOGY vol. 104, no. 4, April 1987, NEW YORK, USA pages 817 - 829 W. EARNSHAW ET AL. 'Molecular cloning of cDNA for CENP-B, the major human centromere autoantigen.'
- CHROMOSOMA vol. 100, no. 6, July 1991, BERLIN, GERMANY pages 360 - 370 K. SULLIVAN ET AL. 'CENP-B is a highly conserved mammalian centromere protein with homology to the helix-loop-helix family of proteins.'
- MOLECULAR BIOLOGY REPORTS vol. 15, no. 3-4, 1991, BOSTON MA, USA page 177 K. SKRINER ET AL. 'Antigenicity of the carboxy-terminus of CENP-B.'
- JOURNAL OF INVESTIGATIVE DERMATOLOGY vol. 97, no. 2, August 1991, BALTIMORE MD, USA pages 378 - 380 Y. MURO ET AL. 'Purification of a human centromere antigen (CENP-B) and application of DNA immunoprecipitation to quantitative assay for anti-CENP-B antibodies.'
- MOLECULAR BIOLOGY REPORTS vol. 16, no. 1, February 1992, BOSTON MA, US pages 49 - 59 R. VERHEIJEN ET AL. 'Molecular cloning of a major CENP-B epitope and its use for the detection of anticentromere autoantibodies.'

## Description

The invention relates to a peptide or fragment thereof which is immunochemically reactive with anticentromere autoantibodies (ACA).

The invention also relates to a method for the detection of anticentromere autoantibodies or detection of a centromere antigen in a test fluid and also to an immunochemical reagent and a test kit for carrying out said detection methods.

Centromere components are one of the targets of a highly selective autoimmune response in certain patients with rheumatic diseases. Although anticentromere autoantibodies (ACA) were observed primarily in limited sclerosis or CREST (Calcinosis, Raynaud's phenomenon, Esophageal dysmotility, Sclerodactyly, Telangiectasia) syndrome, they have also been detected in patients with several other rheumatic diseases and vasculitis.
Earnshaw et al. showed in 1985 that ACA positive patient sera recognize three immunologically related centromere antigens, CENP-A (17kDa), CENP-B (80kDa), and CENP-C (140kDa). Since antibodies to CENP-B were found to be present at high titers in all ACA positive patient sera, whereas the titers of antibodies to CENP-A and CENP-C were often lower, CENP-B was referred to as the major centromere antigen. It has been shown that ACA and antitopoisomerase I antibodies are helpful clinical markers in systemic sclerosis. Earnshaw et al. succeeded in 1987 in cloning the cDNA of ^{~}95% of the mRNA that encodes CENP-B. They also reported the first use of a protein, i.e. CENP-B, in an enzyme-linked immunosorbent assay (ELISA) system to detect autoantibodies in patient sera. The protein used in their experiments encoded 147 amino acid residues from the carboxyl-terminal region of CENP-B fused to 113kDa of β-galactosidase. However, for the development of a specific and sensitive method to enable a reliable diagnosis to be made it is of great importance to identify immuno-dominant epitopes on said protein. The last 60 C-terminal amino acid residues of CENP-B surprisingly constitute an important and unexpected autoantigenic domain. The data show that patient sera in which ACA can be detected by immunofluorescence and immunoblotting all contain antibodies against this C-terminal CENP-B fragment. Furthermore, the expression level of this particular part of CENP-B as fusion protein in E. coli is much higher than the expression level of the last 157 C-terminal amino acid residues of CENP-B, whereas its antigenicity surprisingly appeared to be at least as high as that of the longer CENP-B fragment. Furthermore this material is very suitable as antigen source in an ELISA system for screening patient sera for anti-CENP-B antibodies.

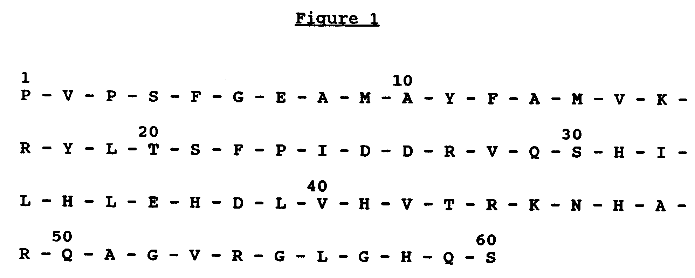

The invention is directed to a polypeptide having the amino acid sequence as shown in figure 1 or a fragment thereof, wherein said polypeptide or fragment thereof are immunochemically reactive with anticentromere autoantibodies.

Analogues or derivatives of the peptide according to Figure 1 are also included in the invention. The term "analogues" refers for instance to post-expression modifications of a peptide, for example, glycosylations, acetylations, phosphorylations etc.

The invention also relates to an immunochemical reagent comprising a polypeptide according to the invention bound to a solid support or provided with a labelling substance.

The invention also comprises a method for the detection of anticentromere autoantibodies in a test fluid, wherein a polypeptide according to the invention is brought into contact with the test fluid and the presence of immune complexes formed between the peptide and antibodies in the test fluid is detected, which is a measure for the presence of anticentromere autoantibodies in the test fluid.

The invention also comprises a method for the detection of centromere antigen in a test fluid, wherein a polypeptide according to the invention is brought into contact with the test fluid and anticentromere autoantibodies, whereafter the presence of immune complexes formed is detected, which is a measure for the presence of centromere antigen in the test fluid.

The invention also relates to a test kit to be used in an immuno-assay, this test kit comprising a polypeptide according to the invention bound to a solid support selected from the group consisting of a microtest well, particles and sols or provided with a labelling substance.

It is within the scope of this invention to use the new nucleotide sequence coding for the amino acids according to Fig. 1 as the basis of a test to detect CENP-B DNA or RNA by a nucleic acid amplification technique for instance the polymerase chain reaction (PCR) or the nucleic acid sequence based amplification (NASBA).

The invention relates to a method for the amplification and the detection of a CENP-B DNA or RNA sequence in a test fluid using a sequence or fragment thereof coding for the amino acid sequence according to the invention as primer(s) in order to perform and to detect a nucleic acid amplification of said CENP-B DNA or RNA sequence.

The invention also relates to a test kit to be used as a test amplification kit for the detection of CENP-B DNA or RNA comprising a nucleic acid sequence or a fragment thereof coding for a polypeptide according to the invention as primer(s).

The peptides mentioned above are found to be particularly suitable for use in a diagnostic method for determining the presence of CENP-B or ACA in a test fluid.

The preparation of the peptides according to the invention is effected by means of one of the known organic chemical methods for peptide synthesis or with the aid of recombinant DNA techniques. This latter method involves the preparation of the desired peptide by means of bringing to expression a recombinant polynucleotide with a polynucleotide sequence which is coding for one or more of the peptides in question in a suitable micro-organism as host.

The organic chemical methods for peptide synthesis are considered to include the coupling of the required amino acids by means of a condensation reaction, either in homogeneous phase or with the aid of a solid phase.

As already indicated above, the peptide according to the invention can likewise be prepared with the aid of recombinant DNA techniques. This possibility is of importance particularly when the peptide is incorporated in a repeating sequence ("in tandem") or when the peptide is prepared as a constituent of a (much larger) protein or polypeptide. This type of preparation of the peptide therefore likewise falls within the scope of the invention.

A polynucleotide of this type, which is coding for the peptide according to the invention, and a recombinant DNA in which this polynucleotide is incorporated likewise fall within the scope of the invention.

Without specifically being incorporated in the claims, it is self-evident that one or more amino acids in the peptides according to the invention can be replaced by other amino acids or amino acid analogues or derivatives without affecting the immunochemical activity of the peptides in question.

Functional derivatives of the above-mentioned peptides viz.:
(a) acid addition salts of the peptides;
(b) amides of the peptides and specifically the C-terminal amides;
(c) esters and specifically C-terminal esters and
(d) N-acyl derivatives, specifically N-terminal acyl derivatives and in particular N-acetyl derivatives, are also considered as peptides according to the invention.

The term "immunochemical reagent" signifies that the peptides according to the invention have been bound to a suitable support or have been provided with a labelling substance.

The supports which can be used are, for example, the inner wall of a microtest well or a cuvette, a tube or capillary, a membrane, filter, test strip or the surface of a particle such as, for example, a latex particle, an erythrocyte, a dye sol, a metal sol or metal compound as sol particle.

Labelling substances which can be used are, inter alia, a radioactive isotope, a fluorescent compound, an enzyme, a dye sol, metal sol or metal compound as sol particle.

In a method for the detection of antibodies directed against a centromere antigen in a test fluid, an immunochemical reagent according to the invention is brought into contact with the test fluid. After which, the presence of immune complexes formed between the peptide and antibodies in the test fluid is a measure for the presence of said antibodies in the test fluid.

The immunochemical reaction which takes place using these detection methods is preferably a sandwich reaction, an agglutination reaction, a competition reaction or an inhibition reaction.

For the detection of a centromere antigen, for instance CENP-B, in a test fluid an immunochemical reagent according to the invention is brought into contact with the test fluid and ACA after which the presence of immune complexes formed is detected and, from this, the presence of CENP-B antigen in a test fluid can be determined.

A particularly suitable method for the detection of CENP-B antigen in a test fluid which can be used is a competition reaction between a peptide according to the invention provided with a labelling substance and a CENP-B antigen (present in the test fluid) for a binding site on the antibody directed against CENP-B which is bound to a solid support.

A test kit according to the invention comprises an immunochemical reagent as described above. For carrying out a sandwich reaction, the test kit may comprise, for example, a peptide according to the invention bound to a solid support, for example the inner wall of a microtest well, and either a labelled synthetic peptide according to the invention or a labelled anti-antibody.

For carrying out a competition reaction, the test kit may comprise a peptide according to the invention bound to a solid support, a labelled antibody directed against CENP-B antigen preferably a monoclonal antibody directed against said peptide then being used to compete with ACA in a test fluid.

In an agglutination reaction an immunochemical reagent which comprises a peptide according to the invention bound to particles including sols must be brought directly into contact with the test fluid in which the antibodies directed against CENP-B antigen which are to be detected are present.

Another embodiment of a test kit is, for example, the use of a labelled peptide according to the invention as immunochemical reagent in a competition reaction with a CENP-B antigen to be detected for a binding site on the antibody directed against CENP-B, which is bound to a solid support.

### Example I

### Sera

Most patient sera were obtained from the Department of Rheumatology of the University Hospital St. Radboud at Nijmegen, The Netherlands. Some additional sera were received from hospitals in Enschede, Deventer and Groningen, The Netherlands.

### Isolation of cDNA clones encoding CENP-B fragments

A mixture of two human sera, both containing anticentromere antibodies, was used to screen a human placental cDNA expression library (Clontech HL100 8b) constructed with the λgt11 vector. Each serum was used at a dilution of 1:1000. To detect specifically bound antibody, ¹²⁵I-labelled sheep anti-human immunoglobulin F(ab)₂ fragment (Amersham, U.K.) was used. By this method, four immunopositive clones were detected. These clones, designated 23 (1009bp), 7(1210bp), 15(1306bp) and 13(970bp) encode 60, 125, 157 and 161 amino acids residues from the carboxyl-terminal region of CENP-B, respectively.

The cDNA's were ligated into plasmids of the pGEX bacterial expression system and fused in phase to genetic sequences encoding glutathione S-transferase (GST; 26kDa). Clone 23 was inserted into the BamHI site of pGEX-2T and clone 15 was inserted into the EcoRI site of pGEX-3X. The fusion proteins produced are named GST-CENP-B/23 (33kDa) and GST-CENP-B/15 (52kDa).

E. coli DH5α was used as host for maintaining the plasmids as well as for the production of fusion proteins.

### DNA sequence analysis

cDNA fragments were digested with a variety of restriction enzymes. DNA fragments were ligated into the polylinker region of M13mp18. Sequence analysis of the DNA fragments was performed by the dideoxy chain termination method.

### Gelelectrophoresis, protein blotting and detection of antigens

SDS-PAGE and transfer of proteins from 13% or 18% polyacrylamide gels onto nitrocellulose sheets (Schleicher & Schuell filters BA85, Dassel, Germany) was performed. The immunoblots were treated and processed. The antibody-antigen complexes were detected with either horseradish peroxidase-conjugated second antibodies or ¹²⁵I-labelled anti-human Ig (whole antibody) from sheep (Amersham).

### Production and purification of fusion proteins

Overnight cultures of E. coli DH5α transformed with pGEX-2T/23 or pGEX-3X/15 were diluted 1:20 in 100 ml of fresh L-broth containing 100 µl/ml ampicillin and grown for 1 hour at 37 °C with intensive aeration before adding IPTG (isopropyl-β-D-thiogalactopyranoside: Research Organics, Cleveland, U.S.A.) to a final concentration of 0.1 mM. After a further 3 hours of growth, cells were pelleted and resuspended in 2 ml phosphate buffered saline (PBS) containing 0.5 mM of the protease inhibitor phenylmethylsulfonyl chloride (PMSC).

Cells were lysed by three times freeze/thawing followed by an incubation with 50 µg/ml deoxyribonuclease I (DNase I) (Sigma) for 20 min at 37 °C. Subsequently, 0.1 mg of lysozyme was added in 0.4 ml of 50% (w/v) sucrose, 10 mM Tris.HCl pH 8.0, 1 mM Na-EDTA pH 8.0 and 100 mM NaCl. Incubation was performed for 30 min on ice. After adding 0.2 ml 10% (v/v) Nonidet P-40 and 0.2 ml Na-EDTA pH 7.5, the suspension was incubated on ice for another 15 min. After adding 3.5 mg/ml Zwittergent detergent 3-14 (Calbiochem, San Diego, U.S.A.), the suspension was sonicated three times 1 min on ice using a Branson sonifier B-12 with microtip. The suspension was layered on a 1 ml 40% (w/v) sucrose solution in PBS and centrifuged for 30 min at 10,000 g and +4 °C. The pellet was then resuspended in 0.5 ml 8M urea. This material was diluted 1:20,000 in coating buffer (Na-carbonate buffer pH 9.6: 35 mM NaHCO₃/15mM Na₂CO₃) for use in the ELISA.

### Example II

### Enzyme-Linked Immunosorbent Assay (ELISA)

For detection of antibodies against CENP-B an enzyme immunoassay was established using Zwittergent purified fusion protein GST-CENP-B/23. Optimal concentrations of antigen, patient sera and conjugates were established after appropriate chess board titrations.

Polystyrene microtiter plates (Greiner; 96 wells) were coated with 100 µl/well of a dilution of GST-CENP-B/23 (0.5 µg/ml) in coating buffer, and kept at +4 °C overnight. The antigen solution was then removed and the plates were washed 5 times with PBS/0.05% (v/v) Tween-20. The remaining free binding sites were blocked by adding 150 µl/well of 0.1% (w/v) bovine serum albumin (BSA) diluted in coating buffer and incubating for 2 hours at room temperature. Thereafter, the plates were washed 5 times with PBS/Tween. The assay was performed with 50 µl serum/well, diluted 1:100, 1:200, and 1:400 in RIA-buffer (0.3% (w/v) BSA, 350mM NaCl, 10mM Tris.HCl pH 7.6, 1% (v/v) Triton X-100, 0.5% (w/v) Na-deoxycholate, 0.1% (w/v) Na-dodecyl sulfate), supplemented with 10% (v/v) normal rabbit serum. The plates were incubated at room temperature for 1 hour, and washed 6 times with PBS/Tween.

To each well 50 µl of one of the following rabbit anti-human peroxidase-conjugated antibodies was added:
- IgG, IgA, IgM, kappa, lambda (DAKOpatts, Glostrup, Denmark: P-212), diluted 1:1000 in RIA-buffer.
- IgG (γ-chains) (DAKOpatts: P-214), diluted 1:5000 in RIA-buffer.
- IgA (α-chains) (DAKOpatts: P-216), diluted 1:2000 in RIA-buffer.
- IgM (µ-chains) (DAKOpatts: P-215), diluted 1:2000 in RIA-buffer.
The plates were incubated for 1 hour at room temperature and washed 6 times with PBS/Tween.

Bound antibodies were visualized with 3,3',5,5'-tetramethylbenzidine (TMB, Sigma) according to standard methods. The staining reaction was stopped after 10 min. by adding 100 µl 2M H₂SO₄/well. Plates were read on a Bio-Rad model 2550 ELISA reader at 450nm. All sera were tested in quadruplicate and the results were averaged. Values higher than twice the level of pooled normal human serum (NS) were considered positive.

The 81 sera, positive for both anti-CENP-A and anti-CENP-B antibodies in the immunoblot analysis, all gave a positive immuno-reaction with the GST-CENP-B/23 protein. Very strong immuno-reactions (OD values >2) were obtained with 65 of the 81 sera. The OD values for the other 16 sera in this group ranged from 0.6 to 2.0.

In conclusion, the ELISA with CENP-B peptide according to the invention may be regarded as an advance in screening patient sera for the presence of ACA.

## Claims

1. A polypeptide having the amino acid sequence as shown in figure 1 or a fragment thereof, wherein said polypeptide or fragment thereof are immunochemically reactive with anticentromere autoantibodies.

2. An immunochemical reagent comprising a polypeptide according to claim 1 bound to a solid support or provided with a labelling substance.

3. A method for the detection of anticentromere autoantibodies in a test fluid, characterised in that a polypeptide according to claim 1 is brought into contact with the test fluid and the presence of immune complexes formed between the peptide and antibodies in the test fluid is detected, which is a measure for the presence of anticentromere autoantibodies in the test fluid.

4. A method for the detection of centromere antigen in a test fluid, characterised in that a polypeptide according to claim 1 is brought into contact with the test fluid and anticentromere autoantibodies, whereafter the presence of immune complexes formed is detected, which is a measure for the presence of centromere antigen in the test fluid.

5. A method for the amplification and the detection of a CENP-B DNA or RNA sequence in a test fluid using a sequence or fragment thereof coding for the amino acid sequence according to claim 1 as primer(s) in order to perform and to detect a nucleic acid amplification of said CENP-B DNA or RNA sequence.

6. Test kit comprising a polypeptide according to claim 1 bound to a solid support selected from the group consisting of a microtest well, particles and sols or provided with a labelling substance.

7. Test amplification kit for the detection of CENP-B DNA or RNA comprising a nucleic acid sequence or a fragment thereof coding for a polypeptide of claim 1 as primer(s).

## Patentansprüche

1. Polypeptid mit einer wie in Figur 1 dargestellten Aminosäuresequenz oder ein Fragment davon, worin genanntes Polypeptid oder Fragment davon mit Anticentromer-Autoantikörpern immunochemisch reagiert.

2. Immunochemisches Reagenz, welches ein Polypeptid gemäss Anspruch 1 umfasst, das an einen festen Träger gebunden ist oder mit einer Markierungssubstanz zur Verfügung gestellt wird.

3. Verfahren zum Nachweis von Anticentromer-Autoantikörpern in einer Testflüssigkeit, dadurch gekennzeichnet, dass ein Polypeptid gemäss Anspruch 1 mit der Testflüssigkeit in Kontakt gebracht wird und die Anwesenheit von Immunkomplexen, die sich zwischen dem Peptid und den Antikörpern in der Testflüssigkeit gebildet haben, nachgewiesen wird, was ein Mass für die Anwesenheit von Anticentromer-Autoantikörpern in der Testflüssigkeit ist.

4. Verfahren zu Nachweis von Centromer-Antigen in einer Testflüssigkeit, dadurch gekennzeichnet, dass ein Polypeptid gemäss Anspruch 1 mit der Testflüssigkeit und Anticentromer-Autoantikörpern in Kontakt gebracht wird, wonach die Anwesenheit von gebildeten Immunkomplexen nachgewiesen wird, was ein Mass für die Anwesenheit von Centromer-Antigen in der Testflüssigkeit ist.

5. Verfahren für die Amplifikation und den Nachweis einer CENP-B DNA- oder RNA-Sequenz in einer Testflüssigkeit unter Verwendung einer Sequenz oder eines Fragments davon, welche(s) die Aminosäuresequenz gemäss Anspruch 1 als Primer codiert, um eine Amplifikation der Nukleinsäure der genannten CENP-B DNA- oder RNA-Sequenz durchzuführen und nachzuweisen.

6. Test-Set, welches ein Polypeptid gemäss Anspruch 1 umfasst, das an einen festen Träger gebunden ist, der aus der Gruppe bestehend aus Microtestplattenvertiefungen, Partikeln oder kolloiden Lösungen (Sol) ausgewählt wird oder der mit einer Markierungssubstanz zur Verfügung gestellt wird.

7. Amplifikationstest-Set zum Nachweis von CENP-B DNA oder RNA, der eine Nukleinsäuresequenz oder ein Fragment davon umfasst, das ein Polypeptid von Anspruch 1 als Primer codiert

## Revendications

1. Polypeptide ayant la séquence d'acides aminés qui est représentée dans la figure 1 ou un fragment de celle-ci, ce polypeptide ou ce fragment de celui-ci étant immunochimiquement réactifs avec des auto-anticorps anticentromères.

2. Réactif immunochimique comprenant un polypeptide suivant la revendication 1, lié à un support solide ou pourvu d'une substance de marquage.

3. Procédé pour la détection d'autoanticorps anticentromères dans un fluide à tester, caractérisé en ce qu'un polypeptide suivant la revendication 1 est mis en contact avec le fluide à tester et que la présence de complexes immuns formés entre le peptide et des anticorps dans le fluide à tester est détectée, ce qui constitue une mesure de la présence d'auto-anticorps anticentromères dans le fluide à tester.

4. Procédé pour la détection d'un antigène de centromère dans un fluide à tester, caractérisé en ce qu'un polypeptide suivant la revendication 1 est mis en contact avec le fluide à tester et des auto-anticorps anticentromères, à la suite de quoi la présence de complexes immuns formés est détectée, ce qui constitue une mesure de la présence d'antigène de centromère dans le fluide à tester.

5. Procédé pour l'amplification et pour la détection d'une séquence d'ADN ou d'ARN de CENP-B dans un fluide à tester utilisant une séquence ou un fragment de celle-ci codant pour la séquence d'acides aminés suivant la revendication 1 en tant qu'amorce(s) pour réaliser et pour détecter une amplification d'acides nucléiques de cette séquence d'ADN ou d'ARN de CENP-B.

6. Kit de test comprenant un polypeptide suivant la revendication 1 lié à un support solide choisi dans le groupe consistant en un puits de microtest, des particules et des sols ou pourvu d'une substance de marquage.

7. Kit d'amplification de test pour la détection d'ADN ou d'ARN de CENP-B comprenant une séquence d'acides nucléiques ou un fragment de celle-ci codant pour un polypeptide suivant la revendication 1 en tant qu'amorce(s).
